# EUROPÄISCHE PATENTANMELDUNG

(11) **EP 4 223 737 A1**
(43) Veröffentlichungstag der Anmeldung: **09.08.2023**
(21) Anmeldenummer: 23152830.8
(22) Anmeldetag: 23.01.2023
(51) Int. Cl.: C07C 45/50, C07C 45/82, C07C 47/02

(54) **OPTIMIERTE THERMISCHE TRENNUNG DURCH VORHERIGEN GASAUSTAUSCH**

(30) Priorität: 08.02.2022 EP 22155546
(71) Anmelder: Evonik Operations GmbH, 45128 Essen (DE)
(72) Erfinder: Brächer, Alexander, 45721 Haltern am See (DE); Markovic, Ana, 45721 Haltern am See (DE); Sale, Anna Chiara, 45657 Recklinghausen (DE); Fridag, Dirk, 45721 Haltern am See (DE); Knossalla, Johannes, 46514 Gahlen (DE); Kucmierczyk, Peter, 44628 Herne (DE); Franke, Robert, 45772 Marl (DE)
(74) Vertreter: Evonik Patent Association

(57) **Zusammenfassung**

Die vorliegende Erfindung betrifft ein Verfahren zur Herstellung von Aldehyden aus C2-bis C20-Olefinen mit einer nachfolgenden thermischen Trennung zur Abtrennung des gebildeten Aldehyds, wobei vor der Membrantrennung ein Gasaustausch durchgeführt wird, mit dem der Partialdruckanteilanteil von Kohlenmonoxid oder Wasserstoff erhöht wird, um die Katalysatorverluste zu verringern.

## Beschreibung

Gegenstand der Erfindung ist ein Verfahren zur Herstellung von Aldehyden aus C2-bis C20-Olefinen mit einer nachfolgenden thermischen Trennung zur Abtrennung des gebildeten Aldehyds, wobei vor der Membrantrennung ein Gasaustausch durchgeführt wird, mit dem der Partialdruckanteilanteil von Kohlenmonoxid oder Wasserstoff erhöht wird, um die Katalysatorverluste zu verringern.

Hydroformylierungsverfahren zur Herstellung von Aldehyden sind dem Fachmann seit langer Zeit bekannt. Dabei werden Olefine mit Synthesegas, einer Mischung aus Kohlenmonoxid (CO) und Wasserstoff (H₂), in Gegenwart eines Katalysatorsystems zu den entsprechenden Aldehyden umgesetzt. Die Hydroformylierung ist ein großtechnisch genutztes Verfahren, welches in Anlagen betrieben wird, die hunderte Kilotonnen (kt) pro Jahr Aldehyd produziert werden können. Dabei typischerweise eingesetzte Katalysatorsysteme sind homogen gelöste Katalysatorsysteme und umfassen Übergangsmetallkomplexe aus zumeist Cobalt oder Rhodium als Metall und phosphorhaltigen Liganden.

Um solche Hydroformylierungsverfahren im Maßstab von hunderten kt wirtschaftlich betreiben zu können kommt es auf verschiedene Faktoren an. Ein wichtiger Faktor ist das Metall des homogen gelösten Katalysatorsystems, da Rhodium und Cobalt bzw. deren als Katalysatorvorstufe eingesetzten Verbindungen relativ hochpreisige Rohstoffe sind. Ein Kernziel beim Betrieb großtechnischer Hydroformylierungsverfahren ist deshalb die Minimierung von Katalysatorverlusten während des Betriebs.

Katalysatorverluste können bei thermischen Trennverfahren auftreten. Thermische Trennung im Sinne der vorliegenden Erfindung meint ein Trennverfahren, bei dem die Trennung anhand des Siedepunktes erfolgt. Thermische Trennverfahren können insbesondere zur Abtrennung der gebildeten Produkte aus der Reaktionslösung eingesetzt werden. Die dabei auftretenden Katalysatorverluste im Prozess können beispielsweise durch Anhaftungen von Clustern in Teilen der Anlage oder durch den Ausfall von unlöslichen Übergangsmetallverbindungen in der Anlage hervorgerufen werden.

Die Aufgabe der vorliegenden Erfindung war deshalb die Bereitstellung eines Verfahrens zur Herstellung von Aldehyden, bei dem die Katalysatorverluste bei der Abtrennung thermische Abtrennung gegenüber bekannten Verfahren verringert werden.

Es hat sich überraschend herausgestellt, dass diese Aufgabe dadurch gelöst werden kann, dass nach der eigentlichen Reaktion, aber vor der thermischen Trennung, ein Gasaustausch durchgeführt wird. Dabei wird das Synthesegas, also eine Mischung, die hauptsächlich Kohlenmonoxid und Wasserstoff umfasst, zumindest partiell gegen Kohlenmonoxid oder Wasserstoff ausgetauscht, wodurch der Partialdruckanteil von Kohlenmonoxid oder Wasserstoff erhöht wird. Der produkthaltige Reaktionsaustrag wird somit im Vergleich zur Reaktion mit einem Gas anderer Zusammensetzung bzw. mit einem Gasgemisch anderer Mischung beaufschlagt, wodurch es in der anschließenden thermischen Trennung zu einer Verringerung des Katalysatorverlustes kommt.

Erfindungsgemäß wird die Aufgabe demnach durch ein Verfahren zur Herstellung von Aldehyden gelöst, wobei das Verfahren zumindest die folgenden Schritte umfasst:
a) Hydroformylierung durch Umsetzung von C2- bis C20-Olefinen mit Synthesegas in Gegenwart eines homogen gelösten Katalysatorsystems, welches mindestens ein Metall aus der Gruppe, bestehend aus Co und Rh, und einen phosphorhaltigen Liganden umfasst, wobei die Hydroformylierung in mindestens einer Reaktionszone und bei einem Druck von 15 bis 350 bar absolut durchgeführt wird, wodurch ein flüssiger Reaktionsaustrag erhalten wird, der zumindest die gebildeten Aldehyde und nicht umgesetzte Olefine enthält und bei dem der Partialdruck von CO oder H₂ maximal 70% des Gesamtgasdrucks, d. h. die Summe der vorliegenden Drücke aller vorhandenen gasförmigen Stoffe ist, ausmacht;
b) Durchführen eines Gasaustauschs am flüssigen Reaktionsaustrag, wodurch ein flüssiger Reaktionsaustrag erhalten wird, bei dem der Partialdruck von CO oder H₂ mehr als 90% des Gesamtgasdrucks, vorzugsweise mehr als 95% des Gesamtgasdrucks, besonders bevorzugt mehr als 98 % des Gesamtgasdrucks ausmacht;
c) ein- oder mehrstufiges Entspannen des aus Schritt b) erhaltenen flüssigen Reaktionsaustrags auf einen Druck von weniger als 1 bar absolut;
d) Zuführen des in Schritt c) entspannten flüssigen Reaktionsaustrags zu einer thermischen Trennung, um den Reaktionsaustrag in eine leichtsiedende und eine hochsiedende Fraktion zu trennen, wobei die leichtsiedende Fraktion zumindest einen Teil der Aldehyde enthält.

Die in dieser Anmeldung genannten Druckwerte sind alle in "bar absolut" angegeben. Bar absolut ist definitionsgemäße eine Angabe des absolut vorliegenden Drucks, d. h. der Druck gegenüber dem Druck Null im leeren Raum (Vakuum).

Schritt a) des erfindungsgemäßen Verfahrens ist die Hydroformylierung der C2-C20-Olefine, vorzugsweise C2- bis C17-Olefine, weiterhin bevorzugt C3 bis C14-Olefine und ganz besonders bevorzugt C6- bis C14-Olefine. Bei dem Verfahren können grundsätzlich die reinen Olefine eingesetzt werden. Eingesetzt werden vorzugsweise jedoch Kohlenwasserstoffströme, die die entsprechenden Olefine enthalten. Die Menge an Olefinen in den Kohlenwasserstoffströmen sollte verständlicherweise ausreichend hoch sein, um eine Hydroformylierung wirtschaftlich betreiben zu können. Bevorzugt enthalten die olefinhaltigen Einsatzgemische praktisch keine weiteren ungesättigten Verbindungen und mehrfach ungesättigte Verbindungen wie Diene oder Acetylenderivate.

Die beim erfindungsgemäßen Verfahren einsetzbaren Kohlenwasserstoffströme können Olefine mit end- und/oder innenständigen C-C-Doppelbindungen umfassen. Die genannten Kohlenwasserstoffströme können außerdem Olefine mit gleicher oder unterschiedlicher Kohlenstoffatomzahl umfassen. Als Olefine eignen sich insbesondere Ethen, Propen, 1- oder 2-Buten oder Mischungen davon, Isobuten, 1- oder 2-Penten oder Mischungen davon, Isopentene, 1-, 2- oder 3-Hexen, 1-Hepten, lineare Heptene mit innenständiger Doppelbindung (2-Hepten, 3-Hepten usw.), Mischungen linearer Heptene, 2- oder 3-Methyl-1-hexen, 1-Octen, lineare Octene mit innenständiger Doppelbindung, Mischungen linearer Octene, 2- oder 3-Methylhepten, 1-Nonen, lineare Nonene mit innenständiger Doppelbindung, Mischungen linearer Nonene, 2-, 3- oder 4-Methyloctene, 1-, 2-, 3-, 4- oder 5-Decen, 2-Ethyl-1-octen, 1-Dodecen, lineare Dodecene mit innenständiger Doppelbindung, Mischungen linearer Dodecene, 1-Tetradecen, lineare Tetradecene mit innenständiger Doppelbindung, Mischungen linearer Tetradecene, 1-Hexadecen, lineare Hexadecene mit innenständiger Doppelbindung und Mischungen linearer Hexadecene.

Propylen wird großtechnisch durch Spaltung von Naphtha hergestellt und ist eine Grundchemikalie, die leicht verfügbar ist. C5-Olefine, also Pentene, sind in Leichtbenzinfraktionen aus Raffinerien oder Crackern enthalten. Technische Gemische, die lineare C4-Olefine, n- und Isobutene, enthalten, sind Leichtbenzinfraktionen aus Raffinerien, C4-Fraktionen aus FC- oder Steamcrackern, Gemische aus Fischer-Tropsch-Synthesen, Gemische aus Dehydrierung von Butanen und durch Metathese oder aus anderen technischen Prozessen entstandene Gemische. Beispielsweise können für das erfindungsgemäße Verfahren geeignete Gemische linearer Butene aus der C4-Fraktion eines Steamcrackers gewonnen werden.

Die höheren Olefine können insbesondere durch Oligomerisierungsreaktionen, beispielsweise Dimerisierung, Trimerisierung oder Tetramerisierung, gewonnen werden. Geeignete Kohlenwasserstoffströme sind weiterhin das bei der Dimerisierung von Propen anfallende Gemisch isomerer Hexene (Dipropen), das bei der Dimerisierung von Butenen anfallende Gemisch isomerer Octene (Dibuten), das bei der Trimerisierung von Propen anfallende Gemisch isomerer Nonene (Tripropen), das bei der Tetramerisierung von Propen oder der Trimerisierung von Butenen anfallende Gemisch isomerer Dodecene (Tetrapropen oder Tributen), das bei der Tetramerisierung von Butenen anfallende isomere Hexadecen (Tetrabuten) sowie durch Cooligomerisierung von Olefinen mit unterschiedlicher Kohlenstoffatomanzahl (bevorzugt 2 bis 4 C-Atome) hergestellte Olefinmischungen, gegebenenfalls nach destillativer Trennung in Fraktionen mit gleicher oder unterschiedlicher C-Zahl. Weiterhin können Olefine oder Olefinmischungen, die durch Fischer-Tropsch-Synthese erzeugt worden sind, eingesetzt werden. Darüber hinaus können Olefine, die durch Olefin-Metathese oder durch andere technische Prozesse hergestellt worden sind, verwendet werden.

Die bei dem Verfahren eingesetzten Olefine werden mit Synthesegas in Gegenwart eines homogen gelösten Katalysatorsystems hydroformyliert. Das molare Verhältnis zwischen Synthesegas und dem Einsatzgemisch sollte zwischen 6:1 und 1:1, vorzugsweise zwischen 3:1 und 1:1, besonders bevorzug zwischen 2:1 und 1:1 liegen. Die Hydroformylierung kann optional in Anwesenheit eines dem Fachmann bekannten Lösemittels durchgeführt wird, vorzugsweise wird jedoch kein Lösemittel verwendet.

Das bei dem erfindungsgemäßen Verfahren eingesetzte homogene Katalysatorsystem umfasst oder besteht aus Co oder Rh, vorzugsweise Rh, und vorzugsweise einem phosphorhaltigen Liganden. In einer besonders bevorzugten Ausführungsform umfasst oder besteht das erfindungsgemäße Katalysatorsystem aus Rh und einem phosphorhaltigen Liganden. Geeignete Liganden für die erfindungsgemäßen Katalysatorsysteme sind dem Fachmann bekannt (siehe z. B. die Lehrbücher "Rhodium Catalyzed Hydroformylation" (aus 2002) von P. W. N. M van Leeuwen oder "Hydrofomylation - Fundamentals, Proceses and Applications in Organic Synthesis" (aus 2016) von A. Börner und R. Franke).

Der phosphorhaltige Ligand für das erfindungsgemäße Katalysatorsystem ist vorzugsweise ein Phosphin (z. B. TPP (Triphenylphosphin)), ein Monophosphit (z. B. Alkanox 240 (Tris(2,4-di-tert-butylphenyl)phosphit) oder ein Bisphosphit (z. B. Biphephos). Es können auch Mischungen von Liganden eingesetzt werden.

Die Hydroformylierung wird vorzugsweise bei den folgenden Bedingungen durchgeführt: Die Temperatur bei der Hydroformylierung liegt vorzugsweise im Bereich von 60 bis 250 °C, weiterhin bevorzugt im Bereich von 70 bis 225 °C und besonders bevorzugt im Bereich von 80 bis 210 °C. Der Druck bei der Hydroformylierung liegt vorzugsweise im Bereich von 10 bis 350 bar absolut, weiterhin bevorzugt im Bereich von 30 bis 325 bar absolut und besonders bevorzugt im Bereich von 45 bis 300 bar absolut.

Die Hydroformylierung wird erfindungsgemäß in mindestens einer Reaktionszone durchgeführt. Eine Reaktionszone umfasst im Sinne der vorliegenden Erfindung mindestens einen Reaktor, in dem die Hydroformylierung durchgeführt wird. Denkbar ist auch, dass die Reaktionszone mehr als einen Reaktor, insbesondere zwei oder drei Reaktoren umfasst, die parallel oder in Reihe geschaltet oder einer Mischform aus paralleler und serieller Schaltung angeordnet sein können.

Der Druck bei der Hydroformylierung entspricht üblicherweise dem Gesamtgasdruck. Der Gesamtgasdruck meint im Rahmen der vorliegenden Erfindung die Summe der vorliegenden Drücke aller vorhandenen gasförmigen Stoffe, also den Druck der (gesamten) Gasphase. Im vorliegenden Verfahren entspricht dies insbesondere der Summe der Partialdrücke von CO und H₂, d. h. der Gesamtgasdruck ist dann der Synthesegasdruck. Bei der erfindungsgemäßen Hydroformylierung macht der Partialdruckanteil von entweder CO oder H₂ maximal 70% des Gesamtgasdrucks, vorzugsweise maximal 65% des Gesamtgasdrucks, besonders bevorzugt maximal 60% des Gesamtgasdrucks aus.

Der bei der Hydroformylierung erhaltene flüssige produkthaltige Reaktionsaustrag steht demnach auch unter dem bei der Hydroformylierung vorliegenden Druck. An dem Reaktionsaustrag wird anschließend in Schritt b) ein Gasaustausch durchgeführt, wodurch ein flüssiger Reaktionsaustrag erhalten wird, bei dem der Partialdruck von CO oder H₂ mehr als 90% des Gesamtgasdrucks, vorzugsweise mehr als 95% des Gesamtgasdrucks, besonders bevorzugt mehr als 98 % des Gesamtgasdrucks ausmacht. Der Partialdruckanteil von entweder CO oder H₂ wird durch diesen Gasaustausch gezielt erhöht, während der Partialdruckanteil von der jeweils anderen Komponente verringert wird. Mögliche Verfahren zum Gasaustausch sind dem Fachmann bekannt.

Der Gasaustausch in Schritt b) erfolgt zum Beispiel dadurch, dass das vorhandene Gas zumindest teilweise abgelassen, d. h. der Reaktionsaustrag entspannt wird, und nachfolgend CO oder H₂ aufgedrückt wird bis der gewünschte Partialdruckanteil an CO oder H₂, also mehr als 90% des Gesamtgasdrucks, vorzugsweise mehr als 95% des Gesamtgasdrucks, besonders bevorzugt mehr als 98 % des Gesamtgasdrucks, vorliegt. Dies kann in einem Schritt oder in mehreren Schritten erfolgen, d. h. das Entspannen des flüssigen Reaktionsaustrags und das nachfolgende Aufdrücken von CO oder H2 erfolgt mehrmals hintereinander. Eine andere Variante ist, dass nach der Entspannung des Austrags aus der Reaktion unter Verwendung einer Mischstrecke und/oder einer Blasensäule mit CO oder H₂ beaufschlagt wird, bis der gewünschte Partialdruckanteil an CO oder H₂, mehr als 90% des Gesamtgasdrucks, vorzugsweise mehr als 95% des Gesamtgasdrucks, besonders bevorzugt mehr als 98 % des Gesamtgasdrucks, vorliegt. Die bei der Entspannung beim Gasaustausch anfallende Gasphase kann zurück zum Reaktor geführt werden.

Das Ablassen des Gases bzw. das Entspannen des flüssigen Reaktionsaustrags und das nachfolgende Aufdrücken von CO oder H₂ ggf. unter Verwendung einer Mischstrecke und/oder einer Blasensäule kann einmal oder mehrmals hintereinander durchgeführt werden, um den gewünschten Partialdruckanteil einzustellen. Nach dem Gasaustausch beträgt der Gesamtgasdruck vorzugsweise 1 bis 70 bar absolut, weiterhin bevorzugt 2 bis 30 bar absolut und besonders bevorzugt 4 bis 20 bar absolut. Nach dem Gasaustausch liegt der Reaktionsaustrag als unter Druck stehende Flüssigkeit vor, wobei der Partialdruckanteil an CO oder H₂ mehr als 90% des Gesamtgasdrucks, vorzugsweise mehr als 95% des Gesamtgasdrucks, besonders bevorzugt mehr als 98 % des Gesamtgasdrucks ausmacht. Der unter Druck stehende Reaktionsaustrag wird dann vorzugsweise für eine gewisse Zeit von mindestens 15 Minuten, besonders bevorzugt mindestens 30 Minuten unter dem Druck mit dem genannten Partialdruckanteil gehalten. Die Temperatur beträgt dabei vorzugsweise 80 °C, besonders bevorzugt 100 °C.

Nach dem Gasaustausch erfolgt in Schritt c) ein einstufiges oder mehrstufiges Entspannen des aus Schritt b) erhaltenen flüssigen Reaktionsaustrags auf einen Druck von weniger als 1 bar absolut, vorzugsweise weniger als 800 mbar absolut. Ziel ist es in diesem Schritt zumindest ein leichtes Vakuum zu erzeugen, um die thermische Trennung zu erleichtern. Das Entspannen von unter Druck stehenden flüssigen Phasen, wie dem aus Schritt b) erhaltenen Reaktionsaustrag, ist dem Fachmann grundsätzlich bekannt. Dafür können beispielsweise geeignete Entspannungsverfahren verwendet werden. Die bei der Entspannung in Schritt c) anfallende Gasphase kann zurück zum Reaktor geführt werden.

Der in Schritt c) entspannte flüssige Reaktionsaustrag wird m nachfolgenden Schritt d) einer thermischen Trennung zugeführt, um den Reaktionsaustrag in eine leichtsiedende und eine hochsiedende Fraktion zu trennen. Die leichtsiedende Fraktion enthält zumindest einen Teil der Aldehyde und kann zusätzlich Alkane und/oder nicht umgesetzte Olefine enthalten. Die hochsiedende Fraktion enthält den Katalysator und kann zusätzlich auch Aldehyde oder hochsiedende Verbindungen enthalten. Thermische Trennung im Sinne der vorliegenden Erfindung meint ein Trennverfahren, bei dem die Trennung anhand des Siedepunktes erfolgt. Entsprechende Verfahren sind dem Fachmann hinlänglich bekannt. In einer bevorzugten Ausführungsform der vorliegenden Erfindung umfasst die thermische Trennung mindestens ein thermisches Trennverfahren umfasst, welches aus der Gruppe, bestehend aus Dünnschichtverdampfung, Destillation, Flashverdampfung, Fallfilmverdampfung und Kurzwegverdampfung, ausgewählt wird. Die thermische Trennung wird vorzugsweise bei einer Temperatur von 20 °C bis 200 °C, bevorzugt zwischen 50 °C und 160 °C durchgeführt.

Das erfindungsgemäße Verfahren kann grundsätzlich weitere Schritte umfassen, sofern der allgemeine Ablauf fundamental erhalten. So ist es beispielsweise möglich, dass vor oder nach der thermischen Trennung mindestens eine Membrantrennung durchgeführt wird. Mit Membrantrennverfahren kann das homogene Katalysatorsystem aus dem restlichen Reaktionsaustrag abgetrennt werden.

Sofern eine Membrantrennung auf die thermische Trennung in Schritt d) folgt, wird die Membrantrennung vorzugsweise bei einem Transmembrandruck von 5 bis 100 bar absolut, weiterhin bevorzugt 10 bis 80 bar absolut und besonders bevorzugt 20 bis 50 bar absolut durchgeführt. Die Membrantrennung wird außerdem in einer bevorzugten Ausführungsform bei einer Temperatur von 0 °C bis 200 °C, weiterhin bevorzugt zwischen 20 °C und 160 °C durchgeführt.

Das bei der Membrantrennung eingesetzte Membranmaterial wird vorzugsweise aus der Gruppe, bestehend aus Poly(dimethyldiloxan)en, Polyimiden, partiell fluorinierten Polymeren, vollständig fluorinierten Polymeren, perfluorinierten Polymeren, amorphen Fluorpolymeren (z.B. Cytop^{®}), amorphen oder teil-kristallinen Perfluoroalkoxypolymeren (z.B. Hyflon^{®}), Block-Copolymeren der vorhergenannten Materialien, Polymeren intrinsicher Mikroporosität (PIM), Poly(aryletherketon)en, insbesondere Poly(etheretherketon)en und Poly(etherketonketon)en, Polybenzimidazolen und Polyethern, ausgewählt. Weiterhin eignen sich Polymere intrinsischer Mikroporosität (PIMs) verschiedenster Monomerzusammensetzung, insbesondere solche die Spirobifluoren-Gruppen enthalten. Das Membranmaterial ist vorzugsweise ein Poly(dimethylsiloxan) (PDMS) oder ein Perfluoroalkoxypolymer wie z.B. Hyflon^{®}.

In einer bevorzugten Ausführungsform ist das verwendete Membranmaterial im Reaktionsaustrag bzw. in dem nach der thermischen Trennung vorliegenden Gemisch intrinsisch stabil, bedarf also keiner weiteren Quervernetzung der Polymerketten mehr.

Es ist weiterhin bevorzugt, wenn auf den aus Schritt d) erhaltenen restlichen Reaktionsaustrag, der an den gebildeten Aldehyden abgereichert worden ist, CO oder H₂ aufgedrückt wird bis der Partialdruck von CO oder H₂ mehr als 90% des Gesamtgasdrucks, vorzugsweise mehr als 95% des Gesamtgasdrucks, besonders bevorzugt mehr als 98 % des Gesamtgasdrucks ausmacht. Da die thermische Trennung bei einem Druck von weniger als 1 bar erfolgt, kann der entsprechende Partialdruckanteil dadurch erreicht werden, dass ein entsprechendes Gas oder Gasgemisch, bei dem der Partialdruckanteil von CO oder H₂ bereits vorliegt, verwendet wird, um es auf den aus Schritt d) erhaltenen restlichen Reaktionsaustrag aufzudrücken.

Das erfindungsgemäße Verfahren kann kontinuierlich oder diskontinuierlich durchgeführt werden. In einer bevorzugten Ausführungsform wird das Verfahren kontinuierlich durchgeführt.

Die vorliegende Erfindung wird nachfolgend anhand von Beispielen erläutert. Diese Beispiele stellen jedoch keine Beschränkung darf, sondern dienen nur der Veranschaulichung.

### Beispiel

### Beispiel 1 - Gasaustausch mit CO

Dicarbonyl(acetylacetonato)rhodium(I) (ca. 200 ppm) als Präkursor für das Übergangsmetall des Katalysatorsystems und der phosphorhaltige Ligand Alkanox^{®} 240 (Tris(2,4-di-tert-butylphenyl)phosphit)) wurden im molaren Verhältnis Rhodium : Ligand von 1 : 20 in 1-Nonanol gelöst und gut vermischt. Von der so hergestellten Lösung wurde eine Probe (1) genommen und auf den Rhodiumgehalt untersucht. Die restliche Lösung wurde in einen Reaktor gegeben mit 25 bar absolut CO für ca. 1 Std. bei 150 °C bedrückt und anschließend ein Vakuum von 300 mbar absolut angelegt. Dabei wurde wieder eine Probe (2) genommen und auf den Rhodiumgehalt untersucht. Der Rhodiumgehalt wurde mittels ICP-MS Analyse (Inductively Coupled Plasma - Mass Spectrometry) bestimmt. Die Ergebnisse sind in Tabelle 1 gezeigt.

**Tabelle 1: Rhodiumgehalt in den Proben 1 und 2**

| **Probe** | **Rhodiumgehalt / ppm** |
|---|---|
| 1 (nach Ansetzen der Lösung) | 195 |
| 2 (nach Aufdrücken von CO und Vakuum) | 191 |

### Beispiel 2 - ohne Gasaustausch

Dicarbonyl(acetylacetonato)rhodium(I) (ca. 200 ppm) als Präkursor für das Übergangsmetall des Katalysatorsystems und der phosphorhaltige Ligand Alkanox^{®} 240 (Tris(2,4-di-tert-butylphenyl)phosphit)) wurden im molaren Verhältnis Rhodium : Ligand von 1 : 20 in 1-Nonanol gelöst und gut vermischt. Von der so hergestellten Lösung wurde eine Probe (3) genommen und auf den Rhodiumgehalt untersucht. Die restliche Lösung wurde in einen Reaktor gegeben mit 25 bar absolut Synthesegas für ca. 1 Std. bei 150 °C bedrückt und anschließend ein Vakuum von 300 mbar absolut angelegt. Dabei wurde wieder eine Probe (4) genommen und auf den Rhodiumgehalt untersucht. Der Rhodiumgehalt wurde mittels ICP-MS Analyse (Inductively Coupled Plasma - Mass Spectrometry) bestimmt. Die Ergebnisse sind in Tabelle 2 gezeigt.

**Tabelle 2: Rhodiumgehalt in den Proben 3 und 4**

| **Probe** | **Rhodiumgehalt / ppm** |
|---|---|
| 3 (nach Ansetzen der Lösung) | 199 |
| 4 (nach Aufdrücken von Synthesegas und Vakuum) | 88 |

Es ist deutlich zu erkennen, dass der Gasaustausch bei nachfolgendem Anlegen eines Vakuums zu einer deutlichen Reduktion des Rhodiumverlustes führt, der Rhodiumgehalt in Probe 2 von Beispiel 1 nachher also deutlicher höher ist als in Probe 4 von Beispiel 2.

### Beispiel 3 - Gasaustausch mit Wasserstoff und Kohlenstoffmonoxid mit einer nachfolgenden Membrantrennung

In diesem Beispiel wurde nach dem Gasaustausch eine Membrantrennung durchgeführt und die Rhodium Konzentration in Permeat und Retentat Strom vermessen, um den Rückhalt des Katalysatorsystems zu bestimmen.

Dicarbonyl(acetylacetonato)rhodium(I) (1,97 g, 7,63 mmol) als Präkursor für das Übergangsmetall des Katalysatorsystems und der phosphorhaltige Ligand Alkanox^{®} 240 (Tris(2,4-di-tert-butylphenyl)phosphit)) (98,74 g, 152,6 mmol) wurden im molaren Verhältnis Rhodium : Ligand von 1 : 20 bei einer Rh-Konzentration von 300 mass-ppm in Toluol (2612,1 g) gelöst.

Mit dieser Lösung wurde eine Membrantrennung in einer Membrantestapparatur mit geschlossenem Kreislauf durchgeführt, wobei Permeat und überschüssiges Retentat wieder in den Zulauf rezykliert werden. Als Membranmaterial wurde kommerziell verfügbare Borsig Membran (Membrantyp - Poly(dimethylsiloxan)) verwendet.

Zunächst wurde der Katalysator bei Synthesegasdruck von 50 bar bei 60 °C im Reaktor ohne Membrantrennung präformiert. Danach wurde der Reaktor auf 20 bar Gesamtgasdruck, und dann anschließend auf 2 bar entspannt. Die Temperatur wurde auf 30 °C gesenkt und die Flachkanaltestzellen in Betrieb genommen. Der Membrankreislauf wurde bei 30 °C sowie 50 bar Feeddruck und 20 bar Permeatvordruck betrieben.

Die Reaktor wurde auf 20 bar Synthesegas gedrückt (Mischung CO : H₂ = ca. 50 : 50). Feed, Retentat und Permeat wurden jeweils Proben genommen und auf ihre Rhodium-Konzentration mittels ICP-MS hin untersucht, um daraus den Rhodium-Rückhalt zu ermitteln (Rhodium-Rückhalt = 1 - (Konzentration Rh im Permeat / Konzentration Rh im Retentat). Der Rhodium-Rückhalt belief sich auf 73% (Messung 1).

Bei laufender Membrantrennung wurde anschließend ein Gaswechsel durchgeführt, in dem der Reaktor mehrmals auf 2 bar entspannt wurde und nachfolgend wieder mit Wasserstoff auf 20 bar aufgedrückt wurde. Dieser Druckwechsel bestehend aus Entspannen und Aufdrücken mit Wasserstoff wurde dreimal wiederholt. Anschließend wurde Messung 2 mit Wasserstoff Feed durchgeführt. Sowohl von Feed als auch von Permeat wurden Proben genommen und auf ihre Rhodium-Konzentration untersucht, um daraus den Rhodium-Rückhalt zu ermitteln. Der Rhodium-Rückhalt belief sich nach dem Aufdrücken von Wasserstoff auf 80%. Es zeigt sich, dass der Rückhalt von Rhodium bei Membrantrennung durch die vorherige Beaufschlagung mit Wasserstoff statt Synthesegas deutlich erhöht werden konnte.

Anschließend wurde ähnlich dem Gaswechsel von Synthesegas auf Wasserstoff der umgekehrte Gaswechsel wieder von Wasserstoff auf Synthesegasdruck durchgeführt, um den Ausgangspunkt wiederherzustellen (Messung 3). Der Rhodium-Rückhalt belief sich hiernach auf 70%.

Hiernach wurde in ähnlicher Weise erneut ein Gaswechsel durchgeführt, wobei diesmal der Gaswechsel auf Kohlenstoffmonoxid durchgeführt wurde (Messung 4). Der Rhodium-Rückhalt belief sich hiernach auf 78 %. Die Ergebnisse sind in Tabelle 3 gezeigt.

**Tabelle 3: Übersicht über den Rückhalt von Rhodium bei den Messungen 1 bis 4**

| Messung | Membranrückhalt Rhodium |
|---|---|
| 1 (nach einsetzen der Lösung und Synthesegas) | 73 % |
| 2 (nach Aufdrücken von Wasserstoff) | 80 % |
| 3 (nach Aufdrucken von Synthesegas) | 70 % |
| 4 (nach Aufdrücken von Kohlenstoffmonoxid) | 78 % |

Es zeigt sich wieder, dass der Rückhalt von Rhodium auch bei nachgeschaltete Membrantrennung durch die vorherige Beaufschlagung mit Kohlenstoffmonoxid oder Wasserstoff statt Synthesegas deutlich erhöht werden konnte. Es lässt sich also zeigen, dass der Gasaustausch sowohl mit Wasserstoff als auch mit Kohlenmonoxid zu einer deutlichen Reduktion des Rhodiumverlustes führt.

## Patentansprüche

1. Verfahren zur Herstellung von Aldehyden, wobei das Verfahren zumindest die folgenden Schritte umfasst:
a) Hydroformylierung durch Umsetzung von C2- bis C20-Olefinen mit Synthesegas in Gegenwart eines homogen gelösten Katalysatorsystems, welches mindestens ein Metall aus der Gruppe, bestehend aus Co und Rh, und einen phosphorhaltigen Liganden umfasst, wobei die Hydroformylierung in mindestens einer Reaktionszone und bei einem Druck von 15 bis 350 bar absolut durchgeführt wird, wodurch ein flüssiger Reaktionsaustrag erhalten wird, der zumindest die gebildeten Aldehyde und nicht umgesetzte Olefine enthält und bei dem der Partialdruck von CO oder H₂ maximal 70% des Gesamtgasdrucks, d. h. die Summe der vorliegenden Drücke aller vorhandenen gasförmigen Stoffe ist, ausmacht;
b) Durchführen eines Gasaustauschs am flüssigen Reaktionsaustrag, wodurch ein flüssiger Reaktionsaustrag erhalten wird, bei dem der Partialdruck von CO oder H₂ mehr als 90% des Gesamtgasdrucks, vorzugsweise mehr als 95% des Gesamtgasdrucks, besonders bevorzugt mehr als 98 % des Gesamtgasdrucks ausmacht;
c) ein- oder mehrstufiges Entspannen des aus Schritt b) erhaltenen flüssigen Reaktionsaustrags auf einen Druck von weniger als 1 bar absolut;
d) Zuführen des in Schritt c) entspannten flüssigen Reaktionsaustrags zu einer thermischen Trennung, um den Reaktionsaustrag in eine leichtsiedende und eine hochsiedende Fraktion zu trennen, wobei die leichtsiedende Fraktion zumindest einen Teil der Aldehyde enthält.

2. Verfahren nach Anspruch 1, wobei die Hydroformylierung in Schritt a) bei einem Druck von 10 bis 350 bar absolut durchgeführt wird.

3. Verfahren nach Anspruch 1 oder 2, wobei das in Schritt a) eingesetzte homogen gelöste Katalysatorsystem mindestens Rh und einen phosphorhaltigen Liganden umfasst.

4. Verfahren nach einem der vorhergehenden Ansprüche, wobei der Gasaustausch in Schritt c) dadurch erfolgt, dass der flüssige Reaktionsaustrag entspannt und nachfolgend CO oder H₂ aufgedrückt wird, bis der gewünschte Partialdruck an CO oder H₂ vorliegt.

5. Verfahren nach Anspruch 4, wobei das Entspannen des flüssigen Reaktionsaustrags und das nachfolgende Aufdrücken von CO oder H₂ mehrmals hintereinander erfolgt.

6. Verfahren nach einem der vorhergehenden Ansprüche, wobei die Hydroformylierung bei einer Temperatur von 80 bis 250 °C durchgeführt wird.

7. Verfahren nach einem der vorhergehenden Ansprüche, wobei der Gesamtgasdruck nach dem Gasaustausch 1 bis 70 bar absolut, vorzugsweise 2 bis 30 bar absolut, vorzugsweise 4 bis 20 bar absolut beträgt.

8. Verfahren nach einem der vorhergehenden Ansprüche, wobei die thermische Trennung bei einer Temperatur von 20 °C bis 200 °C, bevorzugt zwischen 50 °C und 160 °C durchgeführt wird.

9. Verfahren nach einem der vorhergehenden Ansprüche, wobei die thermische Trennung mindestens ein thermisches Trennverfahren umfasst, welches aus der Gruppe, bestehend aus Dünnschichtverdampfung, Destillation, Flashverdampfung, Fallfilmverdampfung und Kurzwegverdampfung, ausgewählt wird.

10. Verfahren nach einem der vorhergehenden Ansprüche, wobei das Verfahren kontinuierlich durchgeführt wird.

11. Verfahren nach einem der vorhergehenden Ansprüche, wobei die Reaktionszone mindestens einen Reaktor umfasst.

12. Verfahren nach einem der vorhergehenden Ansprüche, wobei vor oder nach der thermischen Trennung mindestens eine Membrantrennung durchgeführt wird.

13. Verfahren nach Anspruch 12, wobei nach der thermischen Trennung auf den an den gebildeten Aldehyden abgereicherter flüssiger Reaktionsaustrag CO oder H₂ aufgedrückt wird bis der Partialdruck von CO oder H₂ mehr als 90% des Gesamtgasdrucks, vorzugsweise mehr als 95% des Gesamtgasdrucks, besonders bevorzugt mehr als 98 % des Gesamtgasdrucks ausmacht.

14. Verfahren nach Anspruch 12 oder 13, wobei bei der Membrantrennung ein Membranmaterial, ausgewählt aus der Gruppe, bestehend aus Poly(dimethyldiloxan)en, Polyimiden, partiell fluorinierten Polymeren, vollständig fluorinierten Polymeren, perfluorinierten Polymeren, amorphen Fluorpolymeren (z.B. Cytop), amorphen oder teil-kristallinen Perfluoroalkoxypolymeren (z.B. Hyflon), Block-Copolymeren der vorhergenannten Materialien, Polymeren intrinsicher Mikroporosität (PIM), Poly(aryletherketon)en, insbesondere Poly(etheretherketon)en und Poly(etherketonketon)en, Polybenzimidazolen und Polyethern, eingesetzt wird.

15. Verfahren nach Anspruch 14, wobei ein Membranmaterial verwendet wird, welches im Reaktionsaustrag bzw. in dem nach der thermischen Trennung vorliegenden Gemisch intrinsisch stabil ist, also keiner weiteren Quervernetzung der Polymerketten mehr bedarf.
